# EUROPÄISCHE PATENTSCHRIFT

(11) **EP 0 460 359 B1**
(45) Veröffentlichungstag und Bekanntmachung des Hinweises auf die Patenterteilung: **15.06.1994**
(21) Anmeldenummer: 91103712.5
(22) Anmeldetag: 11.03.1991
(51) Int. Cl.: A01J 5/04, G01N 1/10

(54) **Verfahren und Vorrichtung zur Entnahme einer repräsentativen Milchprobe**
Method and device for taking a representative milk sample
Procédé et dispositif pour le prélèvement d'un échantillon réprésentatif de lait

(30) Priorität: 08.06.1990 DE 4018468
(43) Veröffentlichungstag der Anmeldung: 11.12.1991
(73) Patentinhaber: ULTRAKUST electronic GmbH, 94239 Gotteszell (DE)
(72) Erfinder: Binder, Wilhelm, W-8391 Tiefenbach (DE); Raudszus, Gerhard, Dipl.-Ing., W-8375 Zachenberg (DE)
(74) Vertreter: Heim, Hans-Karl, Dipl.-Ing.

(56) Entgegenhaltungen:
- DE-A- 3 221 222
- DE-A- 3 502 858
- DE-A- 3 528 827
- US-A- 4 795 314
- MACHINE DESIGN Bd. 50, Nr. 25, November 1978, Seiten 131 - 134; G. FRIED: 'PUMPING PROBLEM FLUIDS '

## Beschreibung

Die Erfindung betrifft ein Verfahren zur Entnahme einer repräsentativen Milchprobe gemäß Oberbegriff des Anspruchs 1 und eine Vorrichtung, insbesondere zur Durchführung des Verfahrens, gemäß Oberbegriff des Anspruches 12.

Ein Verfahren und eine Vorrichtung der vorgenannten Art sind z.B. aus der DE 35 02 858 A1 bekannt. Bei dieser bekannten Vorrichtung wird die Milchprobe mittels einer Peristaltikpumpe aktiv aus der Förderleitung in eine Probeflasche gepumpt. Hierbei wird die Peristaltikpumpe so gesteuert, daß entsprechend einer vorher eingegebenen Soll-Menge der Milchcharge die Probeflasche für die Milchprobe entsprechend einem Soll-Volumen gefüllt wird.

Um hierbei ein reproduzierbares Soll-Volumen zu erhalten, ist die Kenntnis der Annahmeleistung des bzw. der Milchsammelfahrzeuge erforderlich. Hierfür wurde bisher ein Parameter fest vorprogrammiert vorgegeben, so daß die für die Aufnahme der Milchprobe bestimmte Probeflasche mehr oder weniger konstant gefüllt wurde.

Da es das Bestreben ist, eine repäsentative Milchprobe für die gesamte Milchcharge zu entnehmen, muß der Entnahmevorgang für die Milchprobe in Abhängigkeit vom Gesamtvolumen der Milchcharge steuerbar sein. Hierbei versteht man unter dem Begriff einer repräsentativen Milchprobe den Sachverhalt, daß einerseits nur ein maximales Volumen in der für die Milchprobe bestimmten Probeflasche zur Verfügung steht, andererseits aber die Milchprobe nach Möglichkeit in etwa ein konstantes Volumen aufweisen sollte, unabhängig davon, ob eine volumenmäßig geringere Milchcharge oder eine sehr große Milchcharge gefördert wird. Das angestrebte konstante Volumen für die Milchprobe resultiert daraus, daß für die Untersuchungen und Tests der Milchprobe bestimmte Mindestmengen erforderlich sind.

Darüber hinaus wird jedoch mit der repräsentativen Milchprobe angestrebt, die Milchprobe über den gesamten Förderungszyklus der entsprechenden Milchcharge zu ziehen. Da jedoch abhängig vom Volumen die Milchchargen mit unterschiedlichen Förderleistungen z.B. aus einem Behälter abgesaugt und in den Milchsammelwagen gepumpt werden, und somit auch unterschiedliche Förderzeiten auftreten, wäre auch dieser Aspekt im Rahmen einer repräsentativen Milchprobe zu berücksichtigen.

Hinzu tritt bei dem Einsatz einer Peristaltikpumpe zur Entnahme einer repräsentativen Milchprobe das Problem, daß das geförderte Volumen nicht im linearen Zusammenhang mit der Umdrehungsgeschwindigkeit der Peristaltikpumpe steht. Nimmt man beispielsweise an, daß eine Peristaltikpumpe bei einer Umdrehungsgeschwindigkeit x1 nach y Umdrehungen das Volumen z1 fördert, so wird üblicherweise das bei einer höheren Geschwindigkeit x2 nach y Umdrehungen geförderte Volumen z2 geringer sein als z1. Dies kann darauf zurückgeführt werden, daß der verwendetete Schlauch durch den Walkprozeß, speziell wenn dieser mit hoher Geschwindigkeit durchgeführt wird, nicht mehr genügend Zeit hat, seinen ursprünglichen Schlauchdurchmesser wieder einzunehmen, um das normale Fassungsvermögen zu erreichen.
Dieser Fehler bringt es daher mit sich, daß bei einem grösseren Volumen einer zu fördernden Milchcharge, deren Förderung mehr Zeit benötigt, wobei kontinuierlich die Milchprobe entnommen werden soll, aufgrund der niedrigeren Umdrehungszahl der Peristaltikpumpe ein höheres als gewünschtes Volumen für die Milchprobe gezogen wird.

Weitere Probleme bei der Förderung einer Milchcharge und bei der Entnahme einer repräsentativen Milchprobe können darin gesehen werden, daß die Förderleistung bzw. Pumpenleistungscharakteristik in der Praxis von Faktoren mit beeinflußt wird, die eine Abweichung von der Soll- oder Nenn-Förderleistung mit sich bringen. Erwähnt seien hierfür z.B. unterschiedliche oder zu enge Querschnitte in der Förderleistung oder im Annahmebereich am Milchtank. Auch kann durch Filterflusen im Sieb der Annahmeleitung die Pumpleistung beeinflußt werden, die gegebenenfalls antriebsbedingt mit der nominell angegebenen Leistung nicht übereinstimmt. Auch die Art der Milchannahme wirkt sich ebenfalls auf die Pumpenleistung aus, wenn z.B. die Milch mit relativ großer Luftbeimengung, insbesondere am Ende der Förderung, aus dem Behälter abgesaugt wird bzw. Behäl ter, die nach unten abgesaugt werden, durch mangelhaftes Aufstellen der Behälter schlecht leerlaufen können. Ebenso kann eine unsachgemäße Handhabung durch den Fahrer bei der Förderung miteingehen.

Derartige Fehlerquellen beeinflussen daher die Förderleistung und Förderzeit für die Übernahme einer Milchcharge aus einem Behälter in einen anderen Behälter.

Der Erfindung liegt daher die A u f g a b e zugrunde, ein Verfahren und eine Vorrichtung der vorgenannten Art so zu verbessern, daß Nachteile der genannten Art vermieden werden und mit Hilfe der Steuerung einer Peristaltikpumpe tatsächlich eine repräsentative Milchprobe auch für unterschiedlichste Volumina der Milchcharge gezogen werden kann.

Diese Aufgabe wird bei einem gattungsgemäßen Verfahren durch die Merkmale des kennzeichnenden Teils des Anspruches 1 und bei einer gattungsgemäßen Vorrichtung durch die Merkmale des kennzeichnenden Teils des Anspruches 12 gelöst.

Die Erfindung berücksichtigt dementsprechend einerseits eine Annahmekennlinie für die Förderleistung einer zum Umpumpen bzw. zum Fördern der Milchcharge eingesetzten Förderpumpe und andererseits eine Pumpenkennlinie für die Peristaltikpumpe, wobei durch diese Pumpenkennlinie Mengenverluste bei höheren Umdrehungsgeschwindigkeiten durch eine entsprechende Erhöhung der Umdrehungszahl n wieder ausgeglichen werden. Bei Ansteuerung der Probenehmer-Einrichtung, die die Peristaltikpumpe aufweist, über die Steuereinrichtung, wird sozusagen eine Korrektur der Umdrehungsgeschwindigkeit der Peristaltikpumpe entsprechend der gespeicherten Pumpenkennlinie durchgeführt.

Weiterhin wird erfindungsgemäß die Förderleistung der Förderpumpe in Abhängigkeit vom Volumen der umzupumpenden oder auzunehmenden Milchcharge entsprechend der Annahme-Kennlinie der Förderpumpe gesteuert.
Um ein effektives Umpumpen bzw. Fördern einer entsprechenden Milchcharge zu gewährleisten, arbeiten neuere Milchsammelwagen in Abhängigkeit vom Volumen der umzupumpenden Milchcharge mit unterschiedlichen Förderleistungen. So kann die Vorrichtung zur Förderung der Milchcharge bei einem Milchsammelwagen bis zur Annahme von 40 l mit einer durchschnittlichen Förderleistung von 230 l/min durchgeführt werden, während z.B. 100 l mit einer Förderleistung von 260 l/min umgepumpt werden und Leistungserhöhungen bis z.B. 320 l/min und höher denkbar sind.

Die Förderleistung beeinflußt daher die Ansteuerung der Peristaltikpumpe, da hierfür nicht nur eine zeitliche sondern auch eine volumenmäßige Abhängigkeit gegeben sein muß.

Die Erstellung der Annahme-Kennlinie wird daher durch unterschiedliche Mengen empirisch ermittelt und ebenfalls abgespeichert. Diese Speicherung erfolgt zweckmäßigerweise in Form von Stützwerten für verschiedene Mengen, z.B. bei einer Milchcharge bis 20 l mit einer Förderleistung von 150 l/min oder bei einer Milchcharge bis 40 l mit einer Förderleistung von 230 l/min.
In Abhängigkeit von der Anzahl der vorgenannten Stützwerte kann daher die Annahme-Kennlinie sehr genau nachgebildet werden.

Geeigneterweise wird das maximal mögliche Volumen der Probeflasche, in die die repräsentative Milchprobe geleitet wird mit einem Überfüllungsgrad von z.B. 120% definiert. Mit anderen Worten wird die Probeflasche normalerweise bei einer Übereinstimmung zwischen Soll-Volumen und Ist-Volumen der zu fördernden Milchcharge lediglich weitgehend, bis z.B. 70 % ihres tatsächlichen Volumens gefüllt. Bei einem erhöhten Ist-Volumen der Milchcharge besteht dann die Möglichkeit, auch für das das Soll-Volumen übersteigende Volumen der Milchcharge bis zu einem vorgebbaren und programmierbaren Überfüllungsgrad die Milchprobe weiter entnehmen zu können, so daß tatsächlich von einer repräsentativen Milchprobe gesprochen werden kann.

Zweckmäßigerweise wird die Entnahme der Milchprobe über Sensoreinrichtungen, die den Beginn der Förderung der Milchcharge und deren Ende erfassen, gesteuert.

Um ein effektives Umpumpen der Milchcharge sicherzustellen, wird vorteilhafterweise mit abgestuften Förderleistungen gearbeitet.
In Fällen, in denen der vorgegebene Überfüllungsgrad der Probeflasche nicht ausreicht und eine weitere Förderung der Milchcharge stattfindet, kann die gezogene Milchprobe nicht mehr als repräsentativ angesehen werden, so daß ein Fehlercode festgestellt wird.

Um praxisbezogene Fehlerquellen, z.B. unterschiedliche Durchmesser bei Förderschläuchen etc. erfassen und eliminieren zu können, wird verfahrens- wie vorrichtungsmäßig die Soll-Förderzeit vorgegeben und mit der Ist-Förderzeit verglichen. Bei Überschreiten der Soll-Förderzeit wird unter Berücksichtigung des Ist-Volumens der Milchcharge ein Fehlercode ausgegeben.

Die erfindungsgemäße Vorrichtung umfaßt mindestens eine Datenerfassungseinrichtung, die mit einer Steuereinrichtung verbunden ist. Die Steuereinrichtung hat die Aufgabe, die Peristaltikpumpe der Probenehmereinrichtung in Abhängigkeit von deren Pumpenkennlinie (P) und der Annahmekennlinie für die Förderung der Milchcharge unter Berücksichtigung der erfaßten Soll- und Ist-Daten zu steuern.
Die Speicherung beider Kennlinien erfolgt dabei zweckmäßigerweise in der Steuereinrichtung selbst, wobei jedoch auch eine dezentrale Speicherung in der Probenehmereinrichtung oder der Datenerfassungseinrichtung für spezielle Zwecke Vorteile mit sich bringt.
Mit Hilfe des erfindungsgemäßen Verfahrens und der entsprechenden Vorrichtung wird daher eine repräsentative Milchprobe erreicht, in der nicht nur Volumenverluste durch die bisher bekannte Steuerung für Peristaltikpumpen sondern auch sich in der Praxis einstellende fehlerhafte Faktoren eliminiert werden können. Darüberhinaus weist das Verfahren und die Vorrichtung auch die Flexibilität auf, Überfüllungsgrade für die Probeflasche selbst zu definieren und abzuspeichern, so daß abgesehen von extremen Abweichungen auch größere Volumina von Milchchargen, die die Soll-Werte erheblich übersteigen, mit einer repräsentativen Milchprobe erfaßt werden können.

Die Erfindung wird nachstehend schematisch anhand eines Blockschaltbildes und zweier Kennlinien noch beispielhaft näher erläutert. Es zeigen:
- Fig. 1: ein Blockschaltbild der Vorrichtung mit den wesentlichen Einrichtungsblöcken;
- Fig. 2: eine Annahmekennlinie A mit Darstellung der Förderleistung in Abhängigkeit von der Menge der Milchcharge, und
- Fig. 3: eine Pumpenkennlinie P als Funktion der Anzahl der Umdrehungen n gegenüber der Umdrehungszahl U/min.

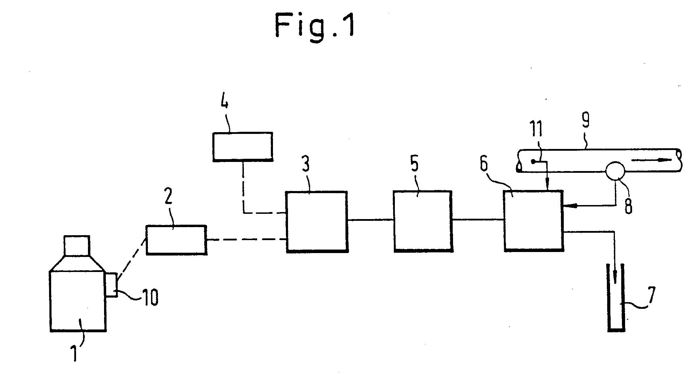

In der Darstellung nach Fig. 1 ist schematisch das Blockschaltbild einer Vorrichtung zur Entnahme einer repräsentativen Milchprobe bei der Förderung einer Milchcharge aus einem Behälter in einen anderen Behälter dargestellt. Die zu fördernde Milchcharge ist dabei in einem Behälter 1 enthalten, der beispielsweise an seiner Außenseite einen Codeträger 10 für die Identifizierung des Lieferanten aufweist. Mittels einer Leseeinrichtung 2 wird dieser Codeträge gelesen und die Daten in eine Datenerfassungs-Einrichtung 3 eingegeben.

Die Datenerfassungs-Einrichtung 3 beinhaltet einen stationären oder mobilen Datenträger 4, der die Soll-Daten für den Lieferanten, z.B. die erwartete Milchmenge gespeichert hat. Dieser Soll-Wert der Milchcharge kann dabei ein automatisch ermittelter Wert einer vorausgegangenen Anzahl von Milchchargen darstellen. Sollte die bedienende Person erkennen, daß der Soll-Wert erheblich vom Ist-Wert der Milchcharge abweicht, so besteht die Möglichkeit, über die Datenerfassungs-Einrichtung 3 manuell einen geänderten SollWert vorzugeben.

Die in der Datenerfassungs-Einrichtung 3 gespeicherten bzw. eingegebenen Daten stehen nunmehr einer Steuereinrichtung 5 zur Verfügung, um die Probenehmer-Einrichtung für eine repräsentative Milchprobe zu steuern.

Die Probenehmer-Einrichtung 6 weist im wesentlichen eine Peristaltikpumpe auf, über die aus einer Leitung 11 die repräsentative Milchprobe in eine Probeflasche 7 gepumpt wird. Darüberhinaus kann auch die Pumpenkennlinie P in der Probenahme-Einrichtung selbst gespeichert sein.

Die Förderung der Milchcharge vom Behälter 1 in einen nicht gezeigten Sammelbehälter, wie z.B. den eines Milchsammelwagens, erfolgt in Pfeilrichtung durch eine Förderleitung 9, an die eine Förderpumpe angeschlossen ist. Die tatsächliche Förderung der Milchcharge, also Beginn und Ende der Förderung werden über einen Sensor 8 überwacht, der mindestens mit der Probenehmer-Einrichtung 6 in Verbindung steht.

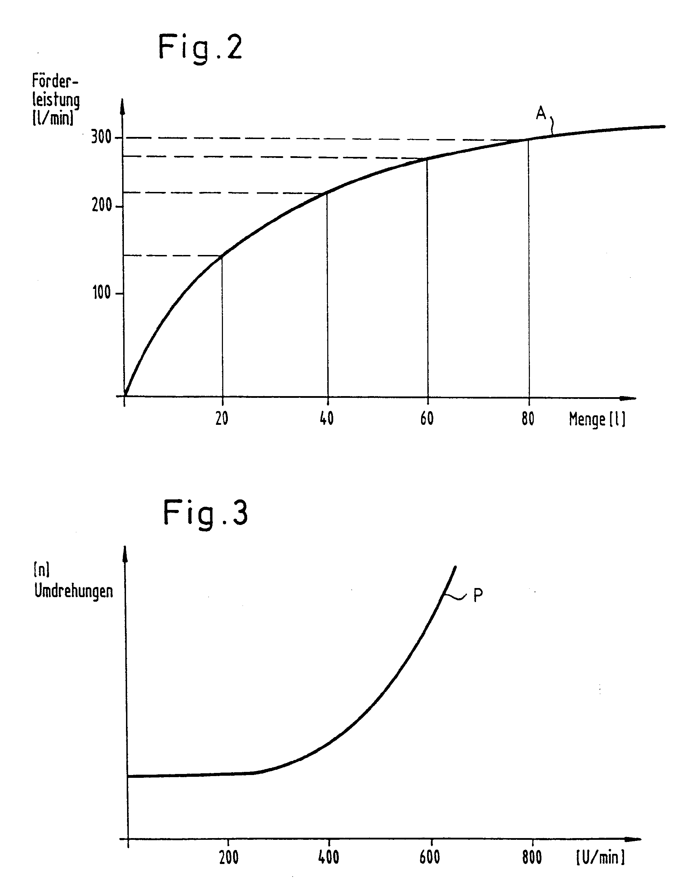

Geht man beispielsweise von einer Milchcharge mit 50 l aus, so arbeitet die Vorrichtung etwa folgendermaßen. In einer Startphase wird die Förderpumpe zum Umpumpen der Milchcharge für die ersten 20 l mit einer Förderleistung von 150 l/min betrieben. Dies entspricht einer Förderzeit von 8 s (Sekunden)(Fig. 2). Die Peristaltikpumpe in der Probenehmer-Einrichtung 6 wird bei Beginn der Förderung der Milchcharge über den Sensor 8 gestartet und läuft mit der entsprechend der korrigierten Pumpenkennlinie P (Fig. 3) mit einer Einstellung von 8 s.

Nach diesem ersten Zeitintervall von 8 Sekunden wird der Steuereinrichtung die nächste Förderleistung entsprechend der Annahmekennlinie, z.B. 230 l/min übergeben, um mit dieser Leistung die nächsten 20 l umzupumpen. Eine Rechnereinheit, die beispielsweise in der Datenerfassungs-Einrichtung 3 angeordnet sein kann, berechnet dementsprechend eine Umpumpzeit von diesmal 5,22 s. Gleichzeitig wird für dieses zweite Intervall die Umdrehungsgeschwindigkeit der Peristaltikpumpe erhöht und für einen Zeitintervall von 5,22 Sekunden betrieben.

Für die restlichen 10 l der umzupumpenden Milchcharge wird die Förderleistung nach Erreichen von 40 l auf 280 l pro Minute angehoben. Dementsprechend ist in einem dritten Intervall ein Betrieb für rein rechnerisch 2,11 Sekunden sowohl der Förderpumpe als auch der Peristaltikpumpe vorgesehen.

Der effektive Stopp der Peristaltikpumpe wird jedoch über den Sensor 8 gesteuert, sobald dieser keine Milch mehr erfaßt bzw. den Sollwert für den Überfüllungsgrad der Probeflasche.

In Fig. 2 ist eine Annahmekennlinie A dargestellt, in der auf der Abszisse die zu fördernde Menge der Milchcharge aufgetragen ist und auf der Ordinate die Förderleistung in l/min. Im Beispiel hat diese Annahmekennlinie A zunächst eine große Steigerung und nähert sich dann bei Mengen von z.B. über 80 l einem asymptotischen Verlauf an.

Als Stützwerte dieser Annahmekennlinie seien folgende Werte genannt. Für 20 l eine Förderleistung von 150 l/min; für 40 l eine Förderleistung von 230 l/min; für 60 l etwa 280 l/min und für 80 l ca. 300 l/min.
Annahmekennlinien dieser Art werden in der Regel empirisch und konstruktionsabhängig erstellt.

In Fig. 3 ist ein möglicher, empirischer Pumpenkennlinienverlauf dargestellt. Die Pumpenkennlinie P gibt die Anzahl der Umdrehungen n (Ordinate) im Vergleich zur Umdrehungsgeschwindigkeit U/min (Abszisse) für ein gleiches, gepumptes Volumen einer Milchprobe wieder. Der Verlauf der Pumpenkennlinie P ist zunächst nahezu geradlinig bis 200 U/min und nimmt dann etwa einen exponentiell ansteigenden Charakter an.

Im Hinblick auf den Füllgrad und den Überfüllungsgrad kann die Vorrichtung z.B. über die Datenerfassungs-Einrichtung so programmiert werden, daß bei der Förderung einer Milchcharge, bei der die Ist-Daten den Soll-Daten entsprechen, der Füllgrad mit 100 % definiert wird, wobei das effektive Volumen der Probeflasche nur zu z.B. 75 % genutzt ist.

Der Prozentsatz der Überfüllung der Probeflasche kann daher eingegeben werden und wird üblicherweise auf 20 % eingestellt. Dementsprechend ist es möglich, die Probeflasche bis zu einem Füllgrad von 120 % mit Milchprobe zu füllen, wobei in diesem Bereich auch übliche Abweichungen des Volumens der Milchcharge liegen. Sollte die Förderung der Milchcharge trotzdem fortgeführt werden und der Füllgrad der Probeflasche bereits bei 120 % liegen, so wird die Probenahme durch die Steuereinrichtung gestoppt und eine Fehlercode für diese Milchprobe abgespeichert. Die Förderung der Milchcharge wird selbstverständlich bis zum Ende durchgeführt.

Bei einer Abweichung des Volumens der Milchcharge nach unten, so daß die Ist-Menge kleiner ist als die Soll-Menge, besteht das vorgenannte Problem zum Füllgrad der Probeflasche nicht. Selbstverständlich könnte auch hier eine "Unterfüllung" als nicht-repräsentativ angesehen werden und eine Fehlermeldung abgespeichert werden. In der Regel jedoch wird die Unterfüllung als akzeptabel und insofern als repräsentativ für die geförderte Milchcharge angesehen.

Die Erfindung erlaubt es daher nach Abschluß der Förderung der betreffenden Milchcharge alle Werte der entsprechenden Milchcharge und der entnommenen repräsentativen Milchprobe zu speichern. Die Möglichkeit eines Ausdruckes kann ebenfalls vorgesehen werden. Zusätzlich zu üblichen Daten wie Lieferanten-Nummer, Datum, Zeit, Temperatur, pH-Wert, Volumen wird auch der erreichte Füllgrad der Probenflasche mit abgespeichert und aufgezeichnet.
Mit der Erfindung können daher Fehlerquellen wie sie üblicherweise bekannt sind, weitgehend ausgeschlossen werden, so daß tatsächlich eine repräsentative Milchprobe aus einer entsprechenden Milchcharge entnommen werden kann.

## Patentansprüche

1. Verfahren zur kontinuierlichen Entnahme einer repräsentativen, volumenmäßig vorgebbaren Milchprobe bei der Förderung einer anzunehmenden Milchcharge eines Lieferanten aus einem Behälter (1) in einen Sammelbehälter durch eine Förderpumpe, bei dem Daten des Lieferanten und Volumen-Soll-Daten der Milchcharge in eine Datenerfassungseinrichtung (3) eingegeben werden und diese Daten einer Steuereinrichtung (5) zugeführt werden, die eine Probenehmer-Einrichtung (6) zur Entnahme der repräsentativen Milchprobe mittels einer Peristaltikpumpe in Abhängigkeit von diesen Daten und gespeicherten Parametern steuert,
dadurch **gekennzeichnet,**
daß die Förderleistung der Förderpumpe in Abhängigkeit von der anzunehmenden Milchcharge als Annahmekennlinie (A) gespeichert wird,
daß die Anzahl der Umdrehungen (n) der Peristaltikpumpe in Abhängigkeit von der Umdrehungsgeschwindigkeit für ein Volumen (z) der zu fördernden Milchprobe als Pumpenkennlinie (P) gespeichert wird,
daß durch die Steuereinrichtung (5) Abhängigkeit vom Volumen der anzunehmenden Milchcharge unter Berücksichtigung der Annahmekennlinie (A) die Förderleistung der Förderpumpe bei der Milchannahme festgelegt wird,
daß danach durch die Steuereinrichtung (5) in Abhängigkeit von der Umdrehungsgeschwindigkeit unter Berücksichtigung der Pumpenkennlinie (P) die Anzahl der Umdrehungen der Peristaltikpumpe gesteuert wird, wobei die Festlegung der Umdrehungsgeschwindigkeit der Peristaltikpumpe in Abhängigkeit von der entsprechend der Annahmekennlinie (A) ausgewählten Förderleistung erfolgt.

2. Verfahren nach Anspruch 1,
dadurch **gekennzeichnet,**
daß die Förderung der Milchcharge mit stufenweiser Änderung der Förderleistung in Abhängigkeit von Soll-daten und gemessenen Ist-Daten der Milchcharge durchgeführt wird.

3. Verfahren nach Anspruch 1 oder 2,
dadurch **gekennzeichnet,**
daß ein Vergleich von Soll- und Ist-Daten bei der Förderung der Milchcharge vorgenommen wird, und
daß bei Überschreiten des definierten Volumenwertes für die repräsentative Milchprobe eine Fehler-Identifizierung erfolgt.

4. Verfahren nach einem der Ansprüche 1 bis 3,
dadurch **gekennzeichnet,**
daß Werte des Füllgrades, z.B. 100 % oder 120 % und der Überfüllungsgrad der Probeflasche in Relation zum effektiven Volumen der Probeflasche eingespeichert werden.

5. Verfahren nach einem der Ansprüche 1 bis 4,
dadurch **gekennzeichnet,**
daß das erwartete Soll-Volumen der zu fördernden Milchcharge manuell in die Datenerfassungs-Einrichtung eingegeben wird.

6. Verfahren nach einem der Ansprüche 1 bis 5,
dadurch **gekennzeichnet,**
daß die Förderleistung für die Förderung der Milchcharge in Abhängigkeit von der Annahmekennlinie und/oder gespeicherten Werten gesteuert wird.

7. Verfahren nach einem der Ansprüche 1 bis 6,
dadurch **gekennzeichnet,**
daß Beginn und Ende der Förderung der Milchcharge sensoriell erfaßt werden und hierüber die Entnahme der Milchprobe gesteuert wird.

8. Verfahren nach einem der Ansprüche 1 bis 7,
dadurch **gekennzeichnet,**
daß die Entnahme der Milchprobe zeitgesteuert für die Dauer der Förderung der Milchcharge durchgeführt wird.

9. Verfahren nach einem der Ansprüche 1 bis 8,
dadurch **gekennzeichnet,**
daß die Soll-Föderleistung für die Förderung der Milchcharge bei der Einstellung der Förderzeit für die Milchcharge gemäß Annahmekennlinie berücksichtigt wird.

10. Verfahren nach einem der Ansprüche 1 bis 9,
dadurch **gekennzeichnet,**
daß jeweils ein weitgehend konstantes Volumen für die Milchprobe bei unterschiedlichen Mengen von Milchchargen entnommen wird.

11. Verfahren nach einem der Ansprüche 1 bis 9,
dadurch **gekennzeichnet,**
daß der Füllgrad der Probeflasche für die zu entnehmende repräsentative Milchprobe in Abhängigkeit von der Ist-Menge der Milchförderung erfolgt.

12. Vorrichtung zur kontinuierlichen Entnahme einer repräsentativen, volumenmäßig vorgebbaren Milchprobe bei der Förderung einer anzunehmenden Milchcharge eines Lieferanten aus einem Behälter (1) in einen Sammelbehälter durch eine Förderpumpe, mit einer Datenerfassung-Einrichtung (3) für die Soll-Daten der Milchcharge und zur Eingabe mindestens von Ist-Daten des Lieferanten und mit einer mit der Datenerfassungs-Einrichtung (3) verbundenen Steuer-Einrichtung (5), mit einer mit einer Peristaltikpumpe ausgestatteten Probenehmer-Einrichtung (6) für die Entnahme der repräsentativen Milchprobe, wobei die Steuer-Einrichtung (5) die Probenehmer-Einrichtung (6) in Abhängigkeit von den erfaßten Daten steuert,
insbesondere zur Durchführung des Verfahrens nach einem der Ansprüche 1 bis 11,
dadurch **gekennzeichnet,**
daß eine Annahmekennlinie (A) für die Förderleistung der Förderpumpe in Abhängigkeit von der anzunehmenden Milchcharge und eine Pumpenkennlinie (P) für die Peristaltikpumpe der Probenehmer-Einrichtung (6) gespeichert sind,
daß als Pumpenkennlinie (P) die Anzahl der Umdrehungen der Peristaltikpumpe als Funktion der Umdrehungsgeschwindigkeit für ein Volumen (z) der zu fördernden Milchprobe gespeichert ist und
daß in Abhängigkeit vom Volumen der anzunehmenden Milchcharge über die entsprechend der Annahme-Kennlinie (A) ausgewählte Förderleistung der Förderpumpe die Umdrehungsgeschwindigkeit der Peristaltikpumpe festlegbar ist und in Abhängigkeit von der Umdrehungsgeschwindigkeit und unter Berücksichtigung der Pumpenkennlinie (P) die Anzahl der Umdrehungen der Peristaltikpumpe durch die Steuereinrichtung (5) steuerbar ist.

13. Vorrichtung nach Anspruch 12,
dadurch **gekennzeichnet,**
daß eine Sensor-Einrichtung (8) zur Feststellung des Beginns und des Endes der Förderung der Milchcharge vorgesehen ist.

14. Vorrichtung nach einem der Ansprüche 12 oder 13,
dadurch **gekennzeichnet,**
daß die Steuer-Einrichtung (5) für eine Förderung der Milchcharge mit abgestufter Förderleistung ausgelegt ist.

## Claims

1. Process for continuously taking a representative milk sample with a predeterminable volume during the delivery of a milk charge to be collected from a supplier from a tank (1) into a collecting tank, in which the supplier data and desired volume data for the milk charge are inputted into a data acquisition unit (3) and said data are supplied to a control means (5), which controls a sampling means (6) for taking the representative milk sample by means of a peristaltic pump, as a fuction of the said data and stored parameters,
**characterized** in
that the delivery capacity of the feed pump is stored as a function of the milk charge to be collected as acceptance characteristic (A),
that the number of revolutions (n) of the peristaltic pump is stored as a function of the speed of revolution for a volume (z) of the milk sample to be delivered as a pumping characteristic (P),
that via the control means (5) as a function of the volume of the milk charge to be collected and while taking account of the acceptance characteristic (A) the delivery capacity of the feed pump is determined during the milk collection,
that then via the control means (5) as a function of the speed of revolution and while taking account of the pumping characteristic (P) the number of revolutions is controlled by the peristaltic pump, the determination of the speed of revolution of the peristaltic pump taking place as a function of the delivery capacity chosen in correspondence with the acceptance characteristic (A).

2. Process according to claim 1,
**characterized** in
that the delivery of the milk charge takes place with stepped modifications of the delivery capacity as a function of desired data and measured actual data the milk charge.

3. Process according to claim 1 or 2,
**characterized** in
that the desired and actual data are compared at the milk charge delivery and if exceeding the defined volume value for the representative milk sample, an error identification takes place.

4. Process according to one of the claims 1 to 3,
**characterized** in
that the values of the degree of filling, e.g. 100% or 120% and the degree of overfilling of the sample bottle are stored in relation to the effective volume of said bottle.

5. Process according to one of the claims 1 to 4,
**characterized** in
that the expected desired volume of the milk charge to be delivered is manually inputted into the data acquisition unit.

6. Process according to one of the claims 1 to 5,
**characterized** in
that the delivery capacity for milk charge delivery is controlled as a function of the acceptance characteristic and/or the stored values.

7. Process according to one of the claims 1 to 6,
**characterized** in
that the start and finish of milk charge delivery are detected by sensors and via this milk sample removal is controlled.

8. Process according to one of the claims 1 to 7,
**characterized** in
that the taking of the milk sample is carried out in time-controlled manner for the duration of the delivery of the milk charge.

9. Process according to one of the claims 1 to 8,
**characterized** in
that the desired delivery capacity for the milk charge delivery is taken into account when setting the feed time for the milk charge in accordance with the acceptance characteristic.

10. Process according to one of the claims 1 to 9,
**characterized** in
that in each case a substantially constant volume for the milk sample is taken at different milk charge quantities.

11. Process according to one of the claims 1 to 9,
**characterized** in
that the degree of filling of the sample bottle for the representative milk sample to be taken takes place as a function of the actual milk delivery quantity.

12. Apparatus for continuously taking a representative milk sample with a predeterminable volume during the delivery of a milk charge to be collected of a supplier from a tank (1) into a collecting tank by means of a feed pump, with a data acquisition unit (3) for the desired data concerning the milk charge and for inputting at least the actual data of the supplier and with a control means (5) connected to the data acquisition unit (3) with a sampling means (6) equipped with a peristaltic pump for taking the representative milk sample, the control means (5) controlling the sampling means (6) as a function of the acquired data, particularly for performing the process according to one of the claims 1 to 11,
**characterized** in
that an acceptance characteristic (A) for the delivery capacity of the feed pump as a function of the milk charge to be collected and a pumping characteristic (P) for the peristaltic pump of the sampling means (6) are stored,
that the number of revolutions of the peristaltic pump is stored as a function of the speed of revolution for a volume (z) of the milk sample to be delivered as pumping characteristic (P) and
that the speed of revolution of the peristaltic pump can be predefined depending on the milk charge volume to be collected via the delivery capacity of the feed pump which has been chosen in accordance with the acceptance characteristic (A) and the number of revolutions of the peristaltic pump can be controlled depending on the speed of revolution and while taking account of the pumping characteristic (P).

13. Apparatus according to claim 12,
**characterized** in
that a sensor means (8) is provided for determining the start and finish of milk charge delivery.

14. Apparatus according to one of the claims 12 or 13,
wherein the control means (5) is designed for a milk charge delivery with stepped delivery capacity.

## Revendications

1. Procédé pour prélever en continu un échantillon représentatif de lait, d'un volume donné, par transfert d'un volume de lait à recevoir d'un fournisseur en provenance d'un réservoir (1) dans un réservoir collecteur par une pompe de refoulement qui délivre les données du fournisseur et les données de consigne de volume dans un dispositif de saisie de donnée commandant un dispositif de prise d'échantillon (6) assurant le prélèvement de l'échantillon représentatif de lait au moyen d'une pompe péristaltique en fonction de ces données et paramètres mis en mémoire, caractérisé en ce que :
- le débit de la pompe refoulante, en fonction de la quantité de lait à recevoir est mis en mémoire sous la forme d'une courbe caractéristique de réception (A),
- le nombre de tours (n) que doit effectuer la pompe péristaltique, en fonction de sa vitesse de rotation pour fournir un volume (Z) d'échantillon de lait est mis en mémoire sous la forme d'une courbe caractéristique de la pompe (P),
- le dispositif de commande (5) détermine le débit de refoulement de la pompe de transfert en fonction de la quantité de lait à recevoir, en utilisant la courbe caractéristique de réception (A),
- le dispositif de commande (5) fait effectuer à la pompe péristaltique le nombre de tours nécessaire, en fonction de sa vitesse de rotation compte tenu de la courbe caractéristique (P), d'où il résulte que la vitesse de rotation de la pompe péristaltique est définie en fonction du débit de refoulement de la pompe choisi sur la courbe caractéristique de réception (A).

2. Procédé selon la revendication 1, caractérisé en ce que le transfert de la quantité de lait est réalisé, avec un débit de refoulement de la pompe variant par étapes en fonction des valeurs de consignes et des valeurs réelles mesurées concernant cette quantité de lait.

3. Procédé selon la revendication 1 ou 2, caractérisé en ce qu'une comparaison est faite entre les valeurs de consigne et les valeurs réelles au cours du refoulement de la quantité de lait et grâce au dépassement de la valeur définie du volume constituant l'échantillon représentatif de lait, une identification d'erreur, se produit.

4. Procédé selon l'une des revendications 1 à 3, caractérisé en ce que les valeurs du degré de remplissage, par exemple 100 % ou 120 % et le degré de suremplissage de la fiole de l'échantillonnage, en relation avec le volume effectif de la fiole, sont mis en mémoire.

5. Procédé selon l'une des revendications 1 à 4, caractérisé en ce que le volume de consigne escompté de la quantité de lait à transférer est introduit manuellement dans le dispositif de saisie des données.

6. Procédé selon l'une des revendications 1 à 5, caractérisé en ce que le débit de refoulement nécessaire au transfert de la quantité de lait est commandé en fonction de la courbe caractéristique de réception et/ou de valeurs mises en mémoire.

7. Procédé selon l'une des revendications 1 à 6, caractérisé en ce que le début et la fin du transfert de la quantité de lait sont déterminés par l'opérateur et commande l'opération de prélèvement de l'échantillon de lait.

8. Procédé selon l'une des revendications 1 à 7, caractérisé en ce que le prélèvement de l'échantillon de lait s'effectue en synchronisme pendant la durée du transfert de la quantité de lait.

9. Procédé selon l'une des revendications 1 à 8, caractérisé en ce que le débit de refoulement de consigne pour le transfert de la quantité de lait est pris en compte dans la détermination de la durée du transfert de la quantité de lait à partir de la courbe caractéristique de réception.

10. Procédé selon l'une des revendications 1 à 9, caractérisé en ce qu'on chaque fois prélève un échantillon de lait de volume constant pour les quantités différentes de lait reçues.

11. Procédé selon l'une des revendications 1 à 9, caractérisé en ce que le degré de remplissage de la fiole contenant l'échantillon représentatif est fonction de la valeur réelle du débit de lait.

12. Dispositif pour prélever en continu un échantillon représentatif de lait, d'un volume donné, par transfert d'un volume de lait à recevoir d'un fournisseur en provenance d'un réservoir (1) dans un réservoir collecteur par une pompe de refoulement comprenant : un dispositif de saisie de données (3), recevant les données de consigne concernant le volume lait et au moins pour l'introduction des données réelles du fournisseur et au dispositif de commande (5) relié au dispositif de saisie de données, un dispositif (6) de prélèvement d'échantillon équipé d'une pompe péristaltique servant à prélever un échantillon représentatif de lait, le dispositif de commande (5) commandant le fonctionnement du dispositif de prélèvement en fonction des données enregistrées, caractérisé en ce que :
- une courbe caractéristique de réception (A), donnant le débit de refoulement de la pompe de transfert en fonction de la quantité de lait à recevoir et une courbe caractéristique (P) de la pompe péristaltique du dispositif de prélèvement de l'échantillon sont mises en mémoire,
- la courbe caractéristique (P) est mise en mémoire en fonction du nombre de tours de la pompe péristaltique nécessaires en fonction de la vitesse de la pompe, pour fournir un volume (Z) d'échantillon,
- la vitesse de rotation de la pompe péristaltique peut être déterminée en fonction du volume de la quantité de lait à recevoir, par l'intermédiaire du débit de refoulement choisi sur la courbe caractéristique de réception (A), puis, en fonction de cette vitesse et compte tenu de la courbe caractéristique (P), le nombre de tours que doit effectuer la pompe péristaltique peut être commandé par le dispositif de commande (5).

13. Dispositif selon la revendication 12, caractérisé en ce qu'il est prévu un dispositif de détection (8) déterminant le début et la fin du transfert de la quantité de lait.

14. Dispositif selon l'une des revendications 12 ou 13, caractérisé en ce que le dispositif de commande (5) du transfert de la quantité de lait procède par débits échelonnés.
